# EUROPEAN PATENT SPECIFICATION

(11) **EP 4 379 386 B1**
(45) Date of publication and mention of the grant of the patent: **19.02.2025**
(21) Application number: 22210333.5
(22) Date of filing: 29.11.2022
(51) Int. Cl.: G01N 35/00, G16H 10/40, G16H 40/20

(54) **METHOD FOR OPERATING AN IN-VITRO-DIAGNOSTICS LABORATORY SYSTEM AND IN-VITRO-DIAGNOSTICS LABORATORY SYSTEM**
VERFAHREN ZUM BETREIBEN EINES IN-VITRO-DIAGNOSTISCHEN LABORSYSTEMS UND IN-VITRO-DIAGNOSTISCHES LABORSYSTEM
PROCÉDÉ DE FONCTIONNEMENT D'UN SYSTÈME DE LABORATOIRE DE DIAGNOSTICS IN VITRO ET SYSTÈME DE LABORATOIRE DE DIAGNOSTICS IN VITRO

(43) Date of publication of application: 05.06.2024
(73) Proprietor: Roche Diagnostics GmbH, 68305 Mannheim (DE); F. Hoffmann-La Roche AG, 4070 Basel (CH)
(72) Inventor: Benton, Daniel, Roxburghshire, TD5 8XX (GB); Ehrenreich, Sebastian, Roxburghshire, TD5 8XX (GB); Hellwig, Marcel, Roxburghshire, TD5 8XX (GB); Toro, Daniel Gomez, Roxburghshire, TD5 8XX (GB)
(74) Representative: Bittner, Thomas L.

(56) References cited:
- US-B2- 11 185 865
- ANONYMOUS: "UniCel DxH 800 - Coulter Cellular Analysis System", BECKMAN COULTER - USER MANUAL, 1 March 2009 (2009-03-01), XP055565251, Retrieved from the Internet <URL:https://www.udh.med.sa/advices/DxH_Operator_Manual.pdf> [retrieved on 20190306]
- SIEMENS HEALTHCARE DIAGNOSTICS PRODUCTS GMBH: "BN ProSpec®System", INSTRUCTION MANUAL - INSTRUCTIONS FOR USE - VERSION 1.4, 1 September 2008 (2008-09-01), XP055455679
- COULTER BECKMAN: "Operations Manual IMMAGE 800 Immunochemistry System For In Vitro Diagnostic Use", 1 September 2016 (2016-09-01), pages 1 - 536, XP093046448, Retrieved from the Internet <URL:https://www.manualslib.com/manual/1470824/Beckman-Coulter-Immage-800.html> [retrieved on 20230512]

## Description

The present disclosure refers to a method for operating an in-vitro-diagnostics (IVD) laboratory system. Further, the present disclosure refers to an IVD laboratory system.

### Background

IVS laboratory systems are known which comprise laboratory devices configured to conduct pre-analysis, analysis, and post-analysis with respect to sample containers receiving a sample to be processed in the laboratory system. Safety mechanisms for operator safety are regularly provided.

For example, document US 6,589,789 B1 relates to an automated device for loading a centrifuge where tubes are presented to the centrifuge via an automated routing system. Safety doors are provided to prevent an operator from reaching into the station. Document US 2009 / 0 180 931 A1 discloses integrated robotic sample transfer devices and components which are used for transferring small samples of material from one registered position to another registered position.

In document EP 2 148 205 B1, a laboratory device for handling sample tubes in the context of storing such sample tubes in a storage compartment as well as an automated tubes identification system are disclosed. Document US 9,316,662 B2 pertains to an automated analyzer including a conveyance mechanism to convey a specimen, an analysis portion to analyze the specimen, and a device cover to cover a movable mechanism including the conveyance mechanism. The automated analyzer is provided with an interlock mechanism and an interlock release mechanism.

Document WO 2018 / 230 198 A1 relates to an automatic analyzer for analyzing component quantities contained in samples such as blood and urine and to an interlock mechanism for performing opening and closing control of a device cover.

The user manual for *"*UniCel DxH 800 Coulter Cellular Analysis System", Beckman Coulter, March 2009, discloses instructions for use of a hematology specimen processing module with system manager. The module has a housing, an actuator, and a cover. When the system massage *"Cover Opened'* occurs, the SPM (specimen processing module) immediately stops operation.

Document US 11 185 865 B2 relates to systems and methods for refrigerated storage of controls and calibrators that utilize a refrigerant storage assembly having an insulated housing and door assembly, thermoelectric coolers, and the refrigerated base assembly having a metal plate thermally coupled to coolers, one or more sensors, and a plurality of receptacles to receive fluid tubes. Refrigerated storage provides a refrigerated environment suitable for multi-day storage of control and calibrator fluids.

### Summary

It is an object of the present disclosure to provide a method for operating an IVD laboratory system as well as an IVD laboratory system with improved safety.

For solving the problem, a method for operating an IVD laboratory system according to independent claim 1 is provided. Further, an IVD laboratory system according to independent claim 15 is provided. Further embodiments are disclosed in dependent claims.

According to one aspect, a method for operating an IVD laboratory system is provided. The IVD laboratory system has a housing with an opening; an actuator device, which is arranged in the housing, for processing sample containers; a cover configured to cover the opening; a cooling device configured to cool the sample containers; and a cooling device control unit configured to control operation of the cooling device. The method comprises: determining whether the cover is open; determining whether the cooling device control unit is active; in a normal system mode, disabling operation of the actuator device based on at least one of the cover being open and the cooling device control unit being inactive; and, in a bypass system mode, enabling operation of the actuator device based on the cover being open and the cooling device control unit being active.

According to another aspect, an IVD laboratory system is provided, comprising: a housing with an opening; an actuator device, which is arranged in the housing, for processing sample containers; a cover configured to cover the opening; a cooling device configured to cool the sample containers; and a cooling device control unit configured to control operation of the cooling device. The system is configured to: determine whether the cover is open; determine whether the cooling device control unit is active; in a normal system mode, disable operation of the actuator device based on at least one of the cover being open and the cooling device control unit being inactive; and, in a bypass system mode, enable operation of the actuator device based on the cover being open and the cooling device control unit being active.

The method may thus comprise disabling operation of the actuator device based on the cover being open and disabling operation of the actuator device based on the cooling device control unit being inactive. The method may comprise determining whether the cover is open or closed and/or determining whether the cooling device control unit is active or inactive.

The method may further comprise, in the normal system mode, enabling operation of the actuator device based on the cover being closed and the cooling device control unit being active and/or, in the bypass system mode, disabling operation of the actuator device based on at least one of the cover being closed and the cooling device control unit being inactive.

The IVD laboratory system may comprise a locking device configured to lock the cover. The method may comprise determining whether the locking device is locked (or unlocked) and/or, in the normal system mode, disabling operation of the actuator device based on the locking device being unlocked. The method may further comprise, in the normal system mode, enabling operation of the actuator device based on the cover being closed, the locking device being locked, and the cooling device control unit being active.

The method may further comprise, in the bypass system mode, disabling operation of the actuator device based on the locking device being locked. The method may comprise, in the bypass system mode, enabling operation of the actuator device based on the cover being open, the locking device being unlocked, and the cooling device control unit being active.

Determining whether the cover is open may be carried out via a cover sensor. The cover sensor may for example be provided in the locking device. The locking device may comprise a locking device sensor configured to determine whether the locking device is locked. The locking device may comprise a solenoid lock.

The method may further comprise switching between the normal system mode and the bypass system mode by a switching device of the IVD laboratory system, preferably by user operation of the switching device of the IVD laboratory system. The switching device may be a key switch, preferably configured to receive a key for switching. The switching device may comprise a normally open output and a normally closed output.

Determining whether the cooling device control unit is active comprises repeatedly transmitting heartbeat signals to a watchdog unit of the IVD laboratory system and/or determining the cooling device control unit as inactive after the time since a last heartbeat signal has been received by the watchdog unit has exceed a timeout threshold.

The timeout threshold may be between 0.1 s and 20 s, preferably between 1 s and 10 s, more preferably between 4 s and 6 s. The watchdog unit may be provided as an integrated circuit and/or a microcontroller.

The heartbeat signals may be transmitted from the cooling device control unit, preferably with a heartbeat signal time interval between 0.1 s and 20 s, preferably between 1 s and 10 s, more preferably between 4 s and 6 s. Additionally or alternatively, the watchdog unit may transmit a probe signal to the cooling device control unit. After receipt of the probe signal, the cooling device control unit may transmit a response signal. The cooling device control unit may then be determined as inactive after the time since the probe signal has been transmitted has exceed a response timeout threshold. The response timeout threshold may be between 0.1 s and 20 s, preferably between 1 s and 10 s, more preferably between 4 s and 6 s.

The method may comprise controlling the cooling device by a second cooling device control unit (different from the cooling device control unit) in reaction to determining the cooling device control unit being inactive. The IVD laboratory system may comprise the second cooling device control unit, which is configured to control operation of the cooling device.

In particular, in reaction to determining the cooling device control unit being inactive, a reset signal may be transmitted from the watchdog unit to a latch, after which the latch preferably transmits a low state output for transfer of control to the second cooling device control unit.

The method may further comprise controlling the cooling device by the cooling device control unit with variable cooling capacity (controlling with cooling capacity adjustment) and/or controlling the cooling device by the second cooling device control unit with fixed cooling capacity.

The fixed cooling capacity may be a maximum cooling capacity of the cooling device. The fixed cooling capacity may also be fixed between 80 % and 100 % of the maximum cooling capacity of the cooling device.

The cooling device may comprise an air-cooling device, e.g., a fan or a plurality of fans. Thus, controlling the cooling device by the cooling device control unit may comprise (variably) controlling a fan speed. Controlling the cooling device by the second cooling device control unit may comprise keeping the fan(s) at a fixed fan speed, e.g., a maximum fan speed.

The method may comprise cooling the sample containers by the cooling device 14, in particular by the fan or the plurality of fans.

The cooling device control unit may be software-implemented and/or the second cooling device control unit may be hardware-implemented.

For example, the cooling device control unit may comprise or be part of a data processing device (with e.g., a general purpose processor) comprising a cooling device control software (in a memory of the data processing system) for controlling operation of the cooling device. The second cooling device control unit may comprise a dedicated control circuit for controlling operation of the cooling device.

Disabling operating of the actuator device may comprise stopping the actuator device in reaction to having determined that the actuator device is at least partially moving. Disabling operating of the actuator device may also comprise stopping a sample container movement in reaction to having determined that the actuator device is moving sample containers. Disabling operating of the actuator device may comprise applying a brake device configured to stop and/or slow and/or prevent (mechanical) movement of/within the actuator device and/or movement of the sample containers.

Disabling operation of the actuator device may comprise interrupting a power supply to the actuator device. Enabling operation of the actuator device may comprise supplying power to the actuator device.

Disabling (enabling) operation of the actuator device may comprise disabling (enabling) an actuator device control unit (actuator device controller) configured to control operation of the actuator device. The actuator device control unit may comprise a plurality of actuator device control subunits, in particular, a motor driver and/or a motor controller. The actuator device control subunits may be implemented as separate integrated circuits (ICs). In particular, a first device control subunit may be motor driver IC, and the second device control subunit may be a motor (servo) controller IC. The first device control subunit and the second device control subunit may be arranged serially.

The actuator device may comprise at least one movable device (movable part), preferably at least one of a sample container transport device, a sample container distribution device, a sample container analysis device, and a motor. The actuator device may in particular comprise at least one of a stepper motor, a servo motor, a rotor, a gripper arm, a conveyor belt, and a magnetic actuator configured to magnetically move sample containers.

The actuator device may be configured to move the sample containers.

The method may comprise, in the normal state mode, disabling operation of the actuator device further based on at least one of the actuator device control unit configured to control operation of the actuator device being in a reset state, the actuator device control unit being in a freeze state, and an emergency user input device being in an active state.

The actuator device control unit may, e.g., comprise motion control chips. The emergency user input device may comprise an emergency button and/or an emergency switch. The emergency user input device may be operable by the user.

The method may comprise disabling the actuator device based on a signal fault from one of the cover, the locking device, the cooling device control unit, the actuator device control unit, and the emergency user input device.

The method may comprise bypassing a signal fault in the locking device signal in the bypass system mode, in particular not disabling the actuator device in reaction to a fault in the locking device signal in the bypass mode. The method may comprise not bypassing the signal fault in the locking device signal in the normal system mode.

The method may further comprise determining the disabling and the enabling of operation of the actuator device using a logical circuit which comprises a cover state, a cooling device control unit state (watchdog control signal for the cooling device), and a system mode state as logical inputs and an actuator device state as logical output.

The logical inputs may comprise logical states (binary states, binary-valued variables). For example, the logical state may be indicative of one of active/inactive, open/closed, activated / not activated, enabled/disabled, and true/false.

The logical inputs may comprise at least one of a controller freeze state of an actuator device control unit configured to control the actuator device, a controller reset state of the actuator device control unit, an emergency user input device state, a cooling device control unit state, a (first and/or second) cover state, a (first and/or second) locking device state, a (first and/or second) bypass state indicative of the system bypass mode, and a safety user input device state.

The logical inputs (input signals) may comprise redundant logical states, preferably for detecting faults in the respective inputting units/devices and/or the wiring (therefrom). For example, a first and a second cover state, and/or a first and a second locking device state, and/or a first and a second bypass state may be provided.

The actuator device may be disabled based on at least one of the first and the second cover state being indicative of the cover being open. The actuator device may also be disabled based on at least one of the first and the locking device state being indicative of the locking device being unlocked.

The actuator device may be disabled based on the first bypass state being different from the second bypass state. In particular, the actuator device may be disabled based on the first bypass state being indicative of a bypass and the second bypass states not being indicative of a bypass. The actuator device may further be disabled based on the first bypass state not being indicative of a bypass and the second bypass states being indicative of a bypass.

The logical circuit may comprise logical gates, in particular at least one of an AND gate, an OR gate, and a NOT gate. The logical inputs may be processed using the logical gates, preferably resulting in the logical output(s).

The logical circuit may be configured to handle single state fault conditions. Operation of the actuator device may be disabled based on single state fault conditions.

The logical circuit may be implemented in an electronic circuit using electronic components. Each logical gate may be implemented using a corresponding electronic component of the electronic circuit. Generally, the method may be carried out in data processing device. The data processing device may also comprise the electronic circuit. The IVD laboratory system may comprise the data processing device and/or the electronic circuit.

The IVD laboratory system may comprise a housing. At least one of or each of the cover, the cooling device, the cooling device control unit, and the locking device may be arranged at or within the housing. The opening may be configured to provide physical user access to the actuator device. The cover may be configured to prevent physical user access to the actuator device. The cover may, e.g., be a door, a window, or a sleeve.

The IVD laboratory system may be configured to test and/or analyze, in particular essentially automatically, samples such as blood or tissue samples that have been taken from the human body, e.g., samples of a bodily fluid. In-vitro-diagnostics can detect diseases or other conditions and can be used to monitor a person's overall health to help cure, treat, or prevent diseases. The IVD laboratory system may be applied in precision medicine to identify patients who are likely to benefit from specific treatments or therapies. In-vitro-diagnostics tests conducted by the IVD laboratory system may be used in laboratory or other health professional settings.

The IVD laboratory system may comprise one or a plurality of IVD instruments. An IVD instrument can be provided with a plurality of IVD components or (e.g., device or instrument) modules. An IVD instrument can be, for example, a pre-analytical, an analytical, and/or a post-analytical IVD instrument. The IVD instrument may be moved and/or controlled by the actuator device. In the IVD laboratory system, the sample containers may be moved along a line of processing for processing. For example, the sample containers may be moved or relocated from a first IVD instrument to a second IVD instrument provided in the line of processing in the IVD laboratory system. The IVD instruments may provide for an IVD working station or location.

A (biological) sample received in a sample container can comprise a biological material, e.g., as taken from a human body or an animal body. A sample can comprise a body fluid, such as blood, interstitial fluid, urine, saliva, or other types of body fluids.

A sample can potentially comprise at least one analyte of interest, e.g. molecules, ions, proteins, metabolites, pathogens, and the like. It may be one of the tasks of IVD testing to detect the presence resp. absence and/or a concentration of one or more analytes in a sample. More generally, IVD testing can refer to determining a biological property of a sample. IVD testing can comprise performing at least one analytical test on a sample, wherein the analytical test can allow to draw conclusions on the biological properties of the sample. The analytical test can, e.g., comprise adding a reagent to the sample, a possible detectable reaction of the sample with the reagent, and / or a detecting or non-detection of this reaction. Detecting of a reaction can, e.g., comprise measuring a physical value of the sample (resp. a composite obtained by using the sample such as a sample-reagent mixture), such as a spectrum and / or an intensity of a radiation reflected by and/or transmitted through the sample (resp. the composite obtained by using the sample).

Processing a sample may, e.g., comprise transporting the sample (typically in sample containers such as IVD tubes; the sample containers may be held in sample container holders such as tube racks), performing pre-analytical steps on the samples (e.g., preparatory steps such as centrifuging), performing analytical steps on the samples (e.g., adding a reagent to the sample and measuring the reaction of the sample with the reagent), and/or performing post-analytical steps on the samples (e.g., storing of a sample in a refrigerator for later use). Processing a sample may comprise one or more physical processing steps (e.g. moving, mixing, heating, etc.).

The embodiments described above in connection with the method for operating the IVD laboratory system may be provided correspondingly for the IVD laboratory system.

### Description of further embodiments

In the following, embodiments, by way of example, are described with reference to figures. In the figures show:
- Fig. 1: graphical representation of an IVD laboratory system;
- Fig. 2: a graphical representation of method for operating an IVD laboratory system;
- Fig. 3: a graphical representation of a truth table for a logical circuit of the IVD laboratory system;
- Fig. 4: a graphical representation of a logical circuit;
- Fig. 5: a graphical representation of a truth table with respect to a normal system mode;
- Fig. 6: a graphical representation of a truth table with respect to a bypass system mode; and
- Fig. 7: a graphical representation of a cooling device control circuit.

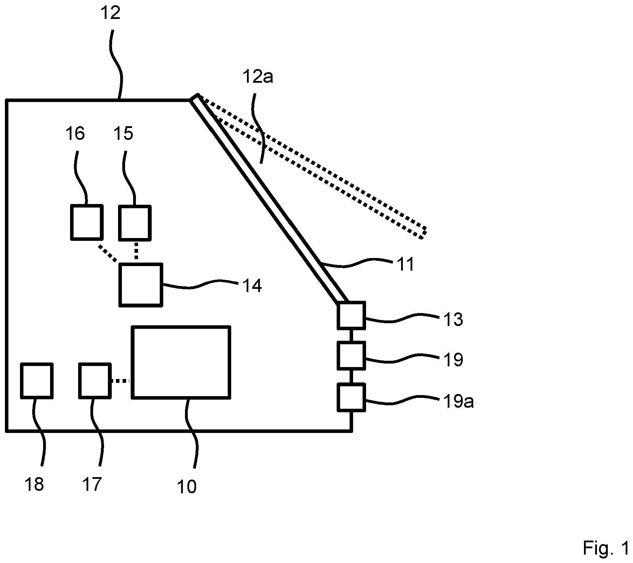

Fig. 1 shows a graphical representation of an IVD laboratory system. The IVD laboratory system comprises an actuator device 10 for processing (e.g., transporting and/or performing (pre-)analysis steps on) sample containers. The sample containers are configured to respectively contain a sample to be processed or handled for at least one of pre-analysis, analysis, and post-analysis in the IVD laboratory system. A cover 11 is provided, e.g., at a housing 12, in order to cover an opening 12a of the housing 12 and/or to cover the actuator device 10. The cover 11 may be in a closed state such that no user (operator) can access the actuator device 10 from outside the housing 12. The cover 11 may also be in an open state (dashed line) for user access of the actuator device 10. A locking device 13 is provided for locking the cover 11, thus preventing the cover 11 from being opened when it is closed. A cooling device (cooler) 14 is provided for cooling the sample containers. A (first) cooling device control unit 15 and a second cooling device control unit 16 are configured to control operation of the cooling device 14. The second cooling device control unit 16 controls the cooling device 14 only in case of the (first) cooling device control unit 15 being inactive.

An actuator device control unit (motion control unit) 17 is configured to control and/or enable and/or disable operation of the actuator device 10. Determining whether the actuator device 10 is to be enabled or disabled may be carried out in a data processing device 18, e.g., an electronic circuit. The actuator device control unit 17 may also be part of the data processing device 18. An emergency user input device 19, e.g., an emergency switch or an emergency button, may be provided. As soon as the emergency user input device is activated (e.g., by pressing the emergency button or by switching the emergency switch), operation of the actuator device 10 is disabled.

Disabling or enabling of the actuator device 10 depends on a system (operation) mode of the IVD laboratory system. In case the IVD laboratory system is in a normal system mode, operation of the actuator device 10 is disabled based on the cover 11 being open or the cooling device control unit 15 being inactive. In other words, in the normal system mode, operation of the actuator device 10 is disabled based on the cover 11 being open and operation of the actuator device 10 is disabled based on the cooling device control unit 15 being inactive.

In contrast, in a bypass system mode, operation of the actuator device 10 is disabled based the cover 11 being closed or the cooling device control unit 15 being inactive. The following table 1 shows the actuator device 10 being enabled or disabled based on different states of the system, the cover 11, and the cooling device control unit 15.

| Table 1 | | | | |
|---|---|---|---|---|
| System mode | Bypass | Cover state | Cooling device control unit state | Actuator device state |
| Normal system mode | Not activated | Open | Inactive | Disabled |
| | Not activated | Open | Active | Disabled |
| | Not activated | Closed | Inactive | Disabled |
| | Not activated | Closed | Active | *Enabled* |
| Bypass system mode | Activated | Open | Inactive | Disabled |
| | Activated | Open | Active | *Enabled* |
| | Activated | Closed | Inactive | Disabled |
| | Activated | Closed | Active | Disabled |

As Table 1 illustrates, the highest priority is assigned to the cooling device control unit state (software state): even when the bypass is activated, the cooling device control unit 15 being inactive (e.g., due to a software fault) will always result in disabling of the actuator device 10. The cover state may also be fixed with a locking device state, i.e., the cover 11 being open may correspond to the locking device 13 being unlocked and the cover 11 being closed may correspond to the locking device 13 being locked. In this case, the cover state also represents the locking device state. When the cover 11 is in a closed state and the bypass is activated, the actuator device 10 is disabled in order to prevent a maintenance key (e.g., for switching between normal system mode and bypass system mode) being left inside the housing 12.

Determining the disabling and the enabling of operation of the actuator device 10 may be carried out using a logical circuit which comprises a plurality of logical (binary) input states and the actuator device state as logical (binary) output state. In particular, eleven input states, comprising a controller freeze state of the actuator device control unit 17, a controller reset state of the actuator device control unit 17, an emergency user input device state, a cooling device control unit state, a first cover state, a second cover state, a first locking device state, a second locking device state, a first bypass state, a second bypass state, and a safety user input device state may be provided.

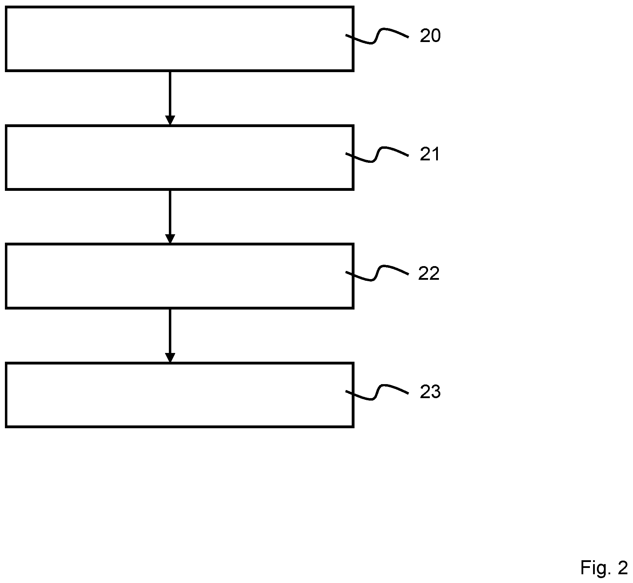

A method for operating a IVD laboratory system may thus comprise the steps shown in Fig. 2. In an initial step 20, the actuator device 10 for processing sample containers, the cover 11 configured to cover the actuator device 10, the cooling device 14 configured to cool the sample containers, and the cooling device control unit 15 configured to control operation of the cooling device 14 are provided. In a first step 21, it is determined whether the cover 11 is open and, in a second step 22, it is determined whether the cooling device control unit 15 is active. In a third step 23, in case the system is in the normal system mode, operation of the actuator device 10 is disabled based on at least one of the cover 11 being open and the cooling device control unit 15 being inactive, and, in case the system is in the bypass system mode, operation of the actuator device 10 is enabled based on the cover 11 being open and the cooling device control unit 15 being active.

The electronic circuit disables the actuator device control units when the cover 11 is open and allows for a bypass for maintenance via a switching device 19a (e.g., a key switch). When the key switch is activated, the IVD laboratory system is in the bypass system mode (maintenance mode). The electronic circuit takes into account the state of the cooling device control unit 15 (software) via a watchdog control signal of a watchdog unit. While the cooling device control unit software is running, the cooling device 14 can be controlled. In case of an error in the cooling device control unit software, the cooling device 14 is driven at maximum cooling capacity (e.g., at full speed of a fan) as a preventive measure against excessive temperature. If the software fails, hardware protection will freeze all motion with respect to the actuator device 10. Further, if the locking device state hardware logic is broken, the cover state may be used and vice versa, which contributes to additional redundancy.

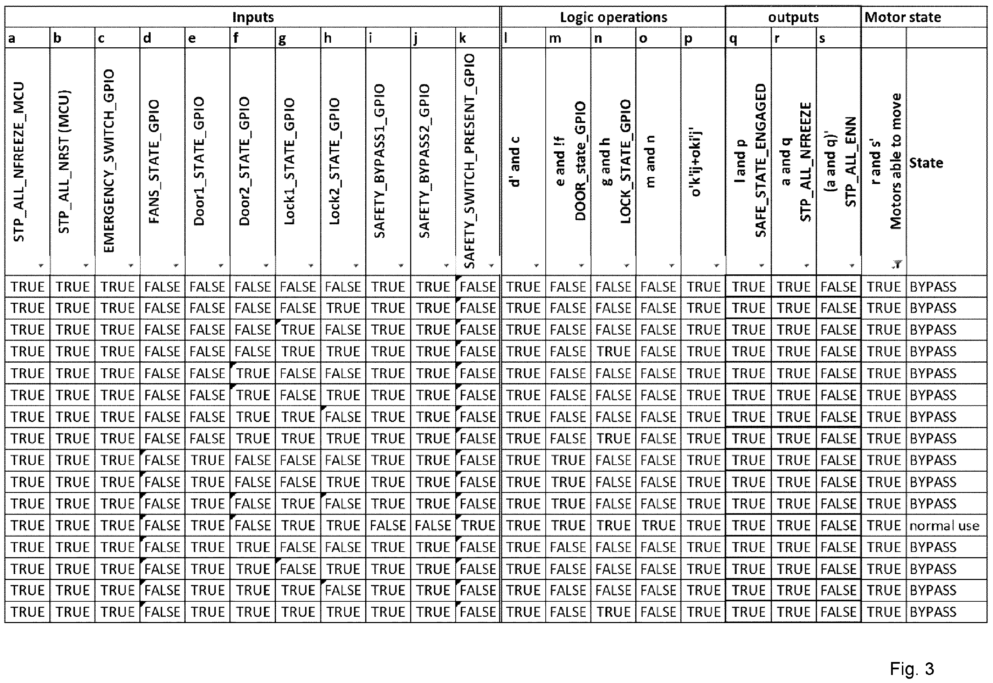

In Fig. 3, showing a truth table for the logical circuit, the input states corresponding to the controller freeze state of the actuator device control unit 17, the controller reset state of the actuator device control unit 17, the emergency user input device state, the cooling device control unit state, the first cover state, the second cover state, the first locking device state, the second locking device state, the first bypass state, the second bypass state, and the safety user input device state are respectively denoted as: STP_ALL_NFREEZE_MCU, STP_ALL_NRST (MCU), EMERGENCY_SWITCH_GPIO, FANS_STATE_GPIO, Door1_STATE_GPIO, Door2_STATE_GPIO, Lock1_STATE_GPIO, Lock2_STATE_GPIO, SAFETY_BYPASS1_GPIO, SAFETY_BYPASS2_GPIO, and SAFETY_SWITCH_PRESENT_GPIO or "a" to "k".

The input states are processed using logical operations resulting in intermediate states "I" to "p" (denoted as "d' and c", "e and !f" / DOOR_state_GPIO, "g and h" / LOCK_STATE_GPIO, "m and n", and "o'k'ij+oki'j'") and output states "q" to "s" (denoted as "I and p" / SAFE_STATE_ENGAGED, "a and q" / STP_ALL_NFREEZE, and "(a and q)'" / STP_ALL_ENN). An STP_ALL_NFREEZE signal may be transmitted to a motor controller IC pin (NFREEZE pin) and an STP_ALL_ENN signal to a motor driver IC pin (ENN pin). STP_ALL_ENN provides an additional signal to disable the actuator device 10 and is used in series with the motor controller IC.

Eleven logical inputs correspond to 2¹¹ possible combinations. Fig. 3 shows relevant input state combinations related to the normal system mode and the bypass system mode in which the actuator device 10 is enabled (see column "motor state" (actuator device state)).

The normal system state in which the motor is additionally enabled (can be actuated) occurs when the actuator device control unit (e.g., motion control chips) are not in reset or freeze state, the software of the cooling device control unit 15 is running, the emergency e-stop button is not pressed, the housing 12 is closed and locked (i.e., the cover 11 is closed and the locking device 13 is locked), and the bypass is not activated. Fig. 3 shows one normal system mode combination and a plurality of bypass system mode combinations. This is due to the bypass resulting in ignoring the state of the locking device 13 being unlocked and the cover 11 being open. When the cover 11 is closed and the bypass is activated, the actuator device 10 is disabled in order to prevent the maintenance key being left inside the housing 12.

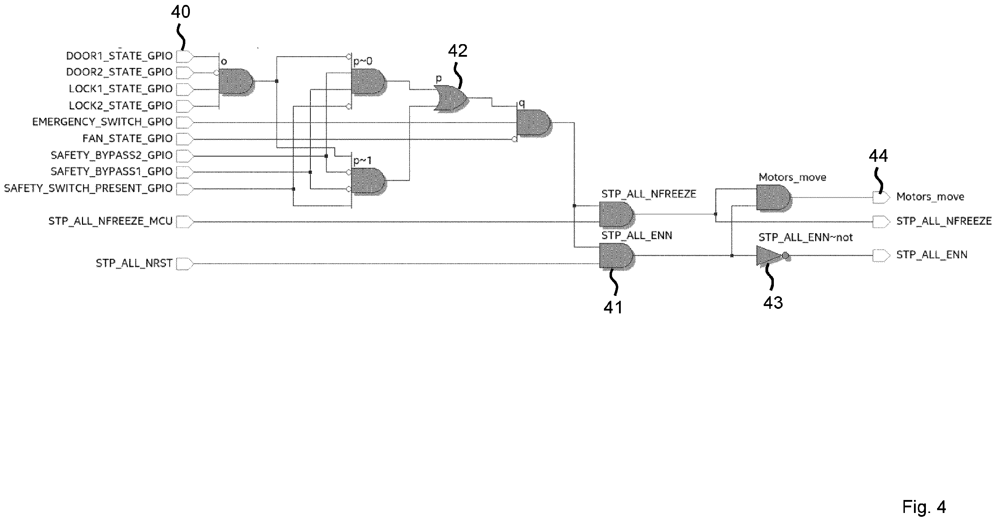

An efficient logical circuit for achieving the truth table according to Fig. 3 is shown in Fig. 4. The input states are respectively provided at input ports 30 and processed in logical gates 41 to 43, which results in the intermediate states. Logical gates 31 correspond to AND gates, logical gate 42 corresponds to an OR gate, and logical gate 43 to a NOT gate. The output states are provided at output ports 44.

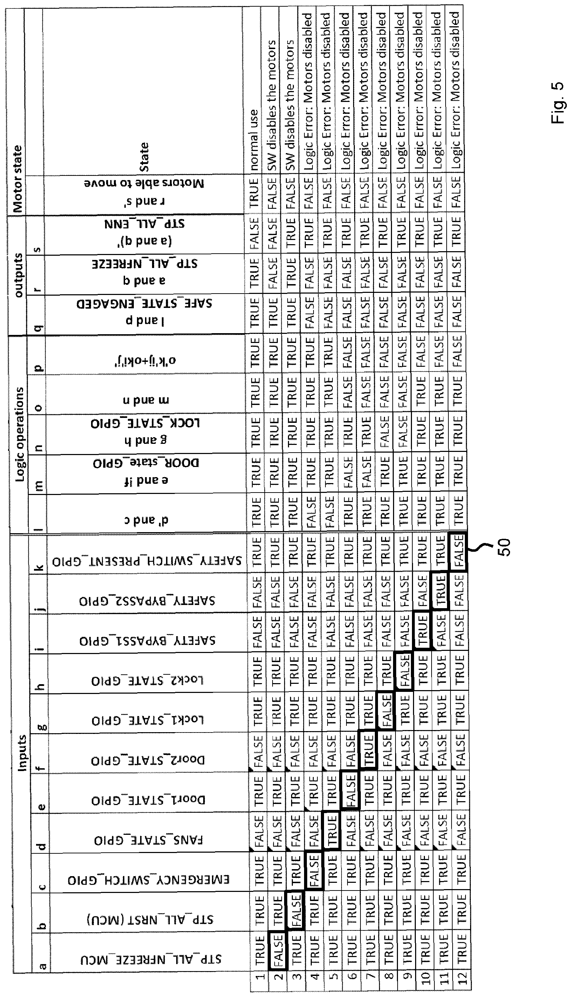

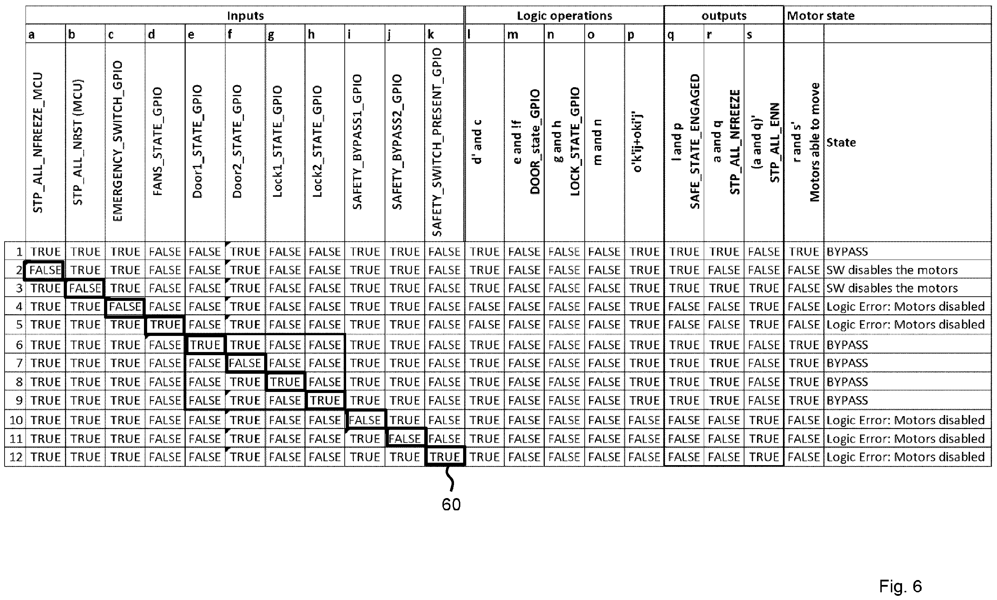

Single fault conditions are handled by the logical circuit as shown in the truth tables according to Fig. 5 (normal state mode) and Fig. 6 (bypass state mode). In particular, Figs. 5 and 6 illustrate different situations and results regarding the enabling (comprising bypassing) or disabling of the actuator device 10 as shown in column "motor state" when an input error (indicated by boxes 50, 60) occurs. In Fig. 5, the normal system state is indicated in the first row and eleven single fault situations (input state combinations) in the remaining rows. Fig. 5 shows that any single fault failure is detected by the logical circuit and the actuator device 10 is thus disabled.

In Fig. 6, the first row corresponds to no fault and the remaining rows to the respective eleven single fault conditions for the bypass system mode. If the cover 11 is open (cf. columns e, f, Door1_STATE_GPIO is false) and any other signal not related to the cover 11 or locking device 13 is faulty (columns a to d and i to k), the actuator device 10 is disabled. A fault in the signals related to the locking device is bypassed. However, when returning to the normal system mode, the signal fault will be detected and the actuator device 10 will be disabled.

The logical circuit may be implemented in an electronic (safety) circuit (e.g., on a printed circuit board, PCB).

In the normal system mode, the inputs logical circuit and the electronic circuit are monitored by software which can disable the actuator device 10 and/or the actuator device control unit 17, in particular, the actuator device control subunits. For example, two actuator device control subunits / motor control integrated circuits (e.g., a motor controller such as Trinamic^{®} TMC4361A and a motor driver such as Trinamic^{®} TMC262) may be disabled to put the actuator device 10 in a safe state. In particular, a TMC4361A motor controller may be disabled via the NFREEZE pin and a TMC262 motor driver via the ENN pin.

The electronic circuit allows for monitoring the input states. The cover 11, the locking device 13, and the bypass signals each have two input states to the electronic circuit which should reflect the same state at all times. Such dual redundant states allow for detection of faults in the wiring of the electronic circuit or the locking device 13. Each redundant input state is monitored by both hardware and software, although both operate independently.

If any of the cover state, the locking device state, and the cooling device control unit state indicates the actuator device 10 should be disabled, the SAFE_STATE_ENGAGED_N signal is activated by the logical circuit. This signal is connected to the same signals used for controlling the actuator device 10, namely NFREEZE (STP_ALL_NFREEZE) and ENN (STP_ALL_ENN). In addition to disabling power of the actuator device 10, a brake is applied, e.g., to a lift axis to prevent the lift axis from dropping when power is disabled.

The electronic circuit ensures that a single fault condition cannot render the IVD laboratory system unsafe or cause a hazard to the operator when the cover 11 is open. In case of no 3.3 V supply, the electronic circuit will not be powered and the actuator device 10 will stop as it will not be supplied with the required control signals.

The switching device may be a key switch configured to receive a key for switching and may, e.g., comprise an Eaton^{®} M22-K01 device. This switch has a normally open output and a normally closed output. When the key switch is activated, a 24 V signal is fed to the inputs of the electronic circuit. Using a normally open contact and a normally closed contact allows for detection of a broken wire.

The contacts of the switching device require a minimum current of 5 mA. Each switching device input is provided with a resistor with 4.3 kΩ. Resistors set the current of each contact to *I_{Switch}* = *U*/*R* = 24 V / 4.3 kΩ = 5.6 mA. A voltage divider (47 kΩ and 7.5 kΩ) generates 3.3 V on a NC7WZ17 input.

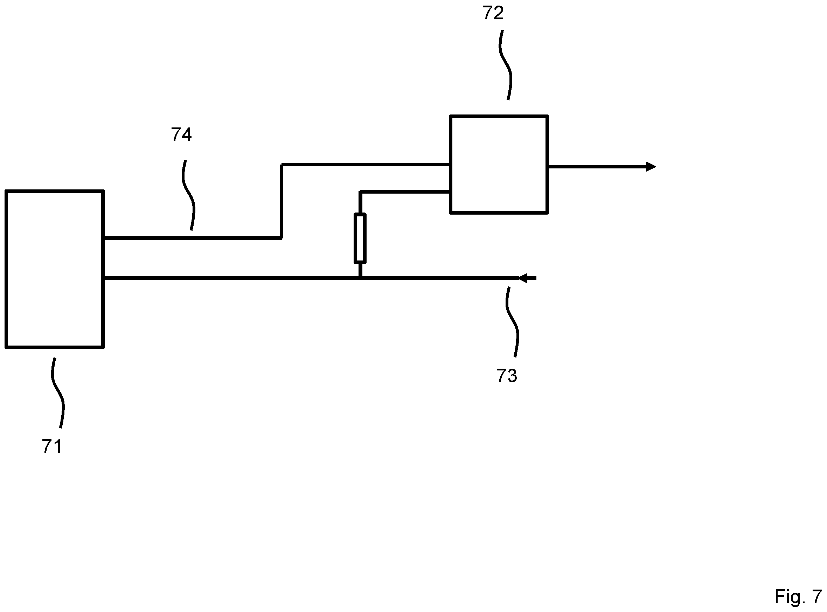

Fig. 7 shows a graphical representation of a cooling device control circuit. Deterioration of the samples in the sample containers can occur if the temperature of the sample is, e.g., more than 5 °C above room temperature. To ensure the temperature is adequately controlled, the cooling device 14, which comprises a plurality of fans, is controlled by the software-implemented (first) cooling device control unit 15 and the second cooling device control unit 16. The speed of the fans is controlled by the software of the cooling device control unit 15. Since the software is not capable of mitigating a hazard, independent hardware (the second cooling device control unit 16) is provided to ensure the fans continue to operate following a software failure. This is achieved via an external watchdog unit 71 and a latch 72.

The software of the cooling device control unit 15 provides a "sign of life" heartbeat signal 73 to the watchdog unit 71, e.g., a watchdog IC, which needs to be provided at least once every 4.9 s. Provided this requirement is met, the software can alter the speed of the fans. During a software failure event, the heartbeat signal will cease to be transmitted and the watchdog unit 71 will produce a reset signal / reset pulse 74. The reset signal 74 is transmitted to a clear input of the latch 72. The heartbeat signal 73 is also connected to a clock input of the latch IC 72. In case the (first) cooling device control unit 15 is active, the heartbeat signal 73 will produce a high output at the latch 72. During a software failure event, the reset pulse 74 from the watchdog unit 71 clears the latch 72 resulting in a low state output from the latch 72. A low output will transfer fan control to the more robust hardware (the second cooling device control unit 16), which will continue to control the fans at full speed until the heartbeat signal 73 reappears. The state of the fan control (cooling device control unit state), hardware or software, is passed to the electronic circuit of the IVD laboratory system.

The locking device 13 may comprise a safety (inter)lock, e.g., an Idec^{®} HS5L-VD7Y4M-G safety lock. The locking device 13 allows for determining whether the cover 11 is open or closed. In particular, a cover position (e.g., door position) may be determined. Further, it may be determined whether the locking device 13 is locked by detecting the state of a solenoid of the locking device 13. When the cover 11 is closed and the locking device 13 is locked, the locking device outputs are 24 V. The solenoid will lock the cover 11 to prevent user access. The user has to request access (via an input device) before the locking device 13 is unlocked. The locking device 13 can for example be unlocked with a software request by the user (e.g., for carrying out preventative maintenance activities). When the locking device 13 is unlocked, the actuator device 10 will be disabled instantaneously.

Table 2 provides an overview of the different statuses of the locking device 13. Status 2 corresponds to a user access request, while the cover 11 (barrier) is in a closed position. In status 4, the locking device 13 is in a locked position, but the cover 11 is open.

| Table 2 | | | | |
|---|---|---|---|---|
| Locking device status | Status 1 | Status 2 | Status 3 | Status 4 |
| Cover state | Closed | Closed | Open | Open |
| Lock state | Locked | Unlocked | Unlocked | locked |
| | Operating status | Safe status | Safe status | Safe status |

## Claims

1. Method for operating an in-vitro-diagnostics laboratory system, IVD laboratory system, the IVD laboratory system having
- a housing (12) with an opening (12a);
- an actuator device (10), arranged in the housing (12), for processing sample containers;
- a cover (11) configured to cover the opening (12a);
- a cooling device (14) configured to cool the sample containers; and
- a cooling device control unit (15) configured to control operation of the cooling device (14);
wherein the method comprises:
- determining whether the cover (11) is open;
- determining whether the cooling device control unit (15) is active;
- in a normal system mode, disabling operation of the actuator device (10) based on at least one of the cover (11) being open and the cooling device control unit (15) being inactive; and
- in a bypass system mode, enabling operation of the actuator device (10) based on the cover (11) being open and the cooling device control unit (15) being active.

2. Method of claim 1, further comprising:
- in the normal system mode, enabling operation of the actuator device (10) based on the cover (11) being closed and the cooling device control unit (15) being active; and
- in the bypass system mode, disabling operation of the actuator device (10) based on at least one of the cover (11) being closed and the cooling device control unit (15) being inactive.

3. Method of claim 1 or 2, wherein the IVD laboratory system comprises a locking device (13) configured to lock the cover (11) and the method comprises:
- determining whether the locking device (13) is locked; and
- in the normal system mode, disabling operation of the actuator device (10) further based on the locking device (13) being unlocked.

4. Method of one of the preceding claims, further comprising switching between the normal system mode and the bypass system mode by user operation of a switching device (19a) of the IVD laboratory system.

5. Method of one of the preceding claims, wherein determining whether the cooling device control unit (15) is active comprises repeatedly transmitting heartbeat signals (73) to a watchdog unit (71) of the IVD laboratory system and determining the cooling device control unit (15) as inactive after the time since a last heartbeat signal has been received by the watchdog unit (71) has exceed a timeout threshold.

6. Method of one of the preceding claims, further comprising controlling the cooling device (14) by a second cooling device control unit (16) in reaction to determining the cooling device control unit (15) being inactive.

7. Method of claim 6, further comprising controlling the cooling device (14) by the cooling device control unit (15) with variable cooling capacity and controlling the cooling device by the second cooling device control unit (16) with fixed cooling capacity.

8. Method of claim 6 or 7, wherein the cooling device control unit (15) is software-implemented and the second cooling device control unit (16) is hardware-implemented.

9. Method of one of the preceding claims, wherein disabling operating of the actuator device (10) comprises stopping the actuator device (10) in reaction to having determined that the actuator device (10) is at least partially moving.

10. Method of one of the preceding claims, wherein disabling operation of the actuator device (10) comprises interrupting a power supply to the actuator device (10).

11. Method of one of the preceding claims, wherein the actuator device (10) comprises at least one of a sample container transport device, a sample container distribution device, a sample container analysis device, and a motor.

12. Method of one of the preceding claims, further comprising, in the normal system mode, disabling operation of the actuator device (10) further based on at least one of an actuator device control unit (17) configured to control operation of the actuator device (10) being in a controller reset state, the actuator device control unit (17) being in a controller freeze state, and an emergency user input device (19) being in an active state.

13. Method of one of the preceding claims, further comprising determining the disabling and the enabling of operation of the actuator device (10) using a logical circuit which comprises a cover state, a cooling device control unit state, and a system mode state as logical inputs and an actuator device state as logical output.

14. Method of claim 13, wherein the logical circuit is implemented in an electronic circuit using electronic components.

15. An in-vitro-diagnostics laboratory system, IVD laboratory system, comprising:
- a housing (12) with an opening (12a);
- an actuator device (10), arranged in the housing (12), for processing sample containers;
- a cover (11) configured to cover the opening (12a);
- a cooling device (14) configured to cool the sample containers; and
- a cooling device control unit (15) configured to control operation of the cooling device (14);
wherein the IVD laboratory system is configured to:
- determine whether the cover (11) is open;
- determine whether the cooling device control unit (15) is active;
- in a normal system mode, disable operation of the actuator device (10) based on at least one of the cover (11) being open and the cooling device control unit (15) being inactive; and
- in a bypass system mode, enable operation of the actuator device (10) based on the cover (11) being open and the cooling device control unit (15) being active.

## Patentansprüche

1. Verfahren zum Betreiben eines In-vitro-Diagnostiklaborsystems, IVD-Laborsystems, wobei das IVD-Laborsystem Folgendes aufweist
- ein Gehäuse (12) mit einer Öffnung (12a);
- eine Aktuatorvorrichtung (10), die in dem Gehäuse (12) angeordnet ist, zum Verarbeiten von Probenbehältern;
- eine Abdeckung (11), die zum Abdecken der Öffnung (12a) ausgebildet ist;
- eine Kühlvorrichtung (14), die zum Kühlen der Probenbehälter ausgebildet ist; und
- eine Kühlvorrichtungssteuerungseinheit (15), die zum Steuern des Betriebes der Kühlvorrichtung (14) ausgebildet ist;
wobei das Verfahren Folgendes umfasst:
- Bestimmen, ob die Abdeckung (11) offen ist;
- Bestimmen, ob die Kühlvorrichtungssteuerungseinheit (15) aktiv ist;
- in einem normalen Systemmodus, Abschalten des Betriebes der Aktuatorvorrichtung (10) basierend darauf, dass die Abdeckung (11) offen ist und/oder die Kühlvorrichtungssteuerungseinheit (15) inaktiv ist; und
- in einem Bypass-Systemmodus, Freigeben des Betriebes der Aktuatorvorrichtung (10) basierend darauf, dass die Abdeckung (11) offen ist und die Kühlvorrichtungssteuerungseinheit (15) aktiv ist.

2. Verfahren nach Anspruch 1, ferner umfassend:
- in dem normalen Systemmodus, Freigeben des Betriebes der Aktuatorvorrichtung (10) basierend darauf, dass die Abdeckung (11) geschlossen ist und die Kühlvorrichtungssteuerungseinheit (15) aktiv ist; und
- in dem Bypass-Systemmodus, Abschalten des Betriebes der Aktuatorvorrichtung (10) basierend darauf, dass die Abdeckung (11) geschlossen ist und/oder die Kühlvorrichtungssteuerungseinheit (15) inaktiv ist.

3. Verfahren nach Anspruch 1 oder 2, wobei das IVD-Laborsystem eine Verriegelungsvorrichtung (13) umfasst, die zum Verriegeln der Abdeckung (11) ausgebildet ist, und das Verfahren Folgendes umfasst:
- Bestimmen, ob die Verriegelungsvorrichtung (13) verriegelt ist; und
- in dem normalen Systemmodus, Abschalten des Betriebes der Aktuatorvorrichtung (10) ferner basierend darauf, dass die Verriegelungsvorrichtung (13) entriegelt ist.

4. Verfahren nach einem der vorstehenden Ansprüche, ferner umfassend das Schalten zwischen dem normalen Systemmodus und dem Bypass-Systemmodus durch Bedienung einer Schaltvorrichtung (19a) des IVD-Laborsystems durch den Benutzer.

5. Verfahren nach einem der vorstehenden Ansprüche, wobei das Bestimmen, ob die Kühlvorrichtungssteuerungseinheit (15) aktiv ist, das wiederholte Übertragen von Herzschlagsignalen (73) an eine Überwachungseinheit (71) des IVD-Laborsystems und das Bestimmen der Kühlvorrichtungssteuerungseinheit (15) als inaktiv umfasst, nachdem die Zeit, zu der ein letztes Herzschlagsignal von der Überwachungseinheit (71) empfangen worden ist, eine Zeitüberschreitungsschwelle überschritten hat.

6. Verfahren nach einem der vorstehenden Ansprüche, ferner umfassend das Steuern der Kühlvorrichtung (14) von einer zweiten Kühlvorrichtungssteuerungseinheit (16) als Reaktion auf das Bestimmen, dass die Kühlvorrichtungssteuerungseinheit (15) inaktiv ist.

7. Verfahren nach Anspruch 6, ferner umfassend das Steuern der Kühlvorrichtung (14) von der Kühlvorrichtungssteuerungseinheit (15) mit variabler Kühlleistung und das Steuern der Kühlvorrichtung von der zweiten Kühlvorrichtungssteuerungseinheit (16) mit festgelegter Kühlleistung.

8. Verfahren nach Anspruch 6 oder 7, wobei die Kühlvorrichtungssteuerungseinheit (15) softwareimplementiert ist und die zweite Kühlvorrichtungssteuerungseinheit (16) hardwareimplementiert ist.

9. Verfahren nach einem der vorstehenden Ansprüche, wobei das Abschalten des Betriebes der Aktuatorvorrichtung (10) das Stoppen der Aktuatorvorrichtung (10) als Reaktion darauf, dass bestimmt wurde, dass die Aktuatorvorrichtung (10) sich mindestens teilweise bewegt, umfasst.

10. Verfahren nach einem der vorstehenden Ansprüche, wobei das Abschalten des Betriebes der Aktuatorvorrichtung (10) das Unterbrechen einer Stromzufuhr zu der Aktuatorvorrichtung (10) umfasst.

11. Verfahren nach einem der vorstehenden Ansprüche, wobei die Aktuatorvorrichtung (10) mindestens eines von einer Probenbehältertransportvorrichtung, einer Probenbehälterverteilungsvorrichtung, einer Probenbehälteranalysevorrichtung und einem Motor umfasst.

12. Verfahren nach einem der vorstehenden Ansprüche, ferner umfassend in dem normalen Systemmodus das Abschalten des Betriebes der Aktuatorvorrichtung (10) ferner basierend darauf, dass sich eine Aktuatorvorrichtungssteuerungseinheit (17), die zum Steuern des Betriebes der Aktuatorvorrichtung (10) ausgebildet ist, in einem Steuerungs-Resetzustand befindet und/oder sich die Aktuatorvorrichtungssteuerungseinheit (17) in einem Steuerungs-Einfrierzustand befindet und/oder sich eine Notfalleingabevorrichtung für Benutzer (19) in einem aktiven Zustand befindet.

13. Verfahren nach einem der vorstehenden Ansprüche, ferner umfassend das Bestimmen des Abschaltens und des Freigebens des Betriebes der Aktuatorvorrichtung (10) unter Verwendung einer Logikschaltung, die einen Abdeckungszustand, einen Kühlvorrichtungssteuerungseinheitszustand und einen Systemmoduszustand als logische Eingaben und einen Aktuatorvorrichtungszustand als logische Ausgabe umfasst.

14. Verfahren nach Anspruch 13, wobei die Logikschaltung unter Verwendung elektronischer Komponenten in eine elektronische Schaltung implementiert wird.

15. In-vitro-Diagnostiklaborsystem, IVD-Laborsystem, umfassend:
- ein Gehäuse (12) mit einer Öffnung (12a);
- eine Aktuatorvorrichtung (10), die in dem Gehäuse (12) angeordnet ist, zum Verarbeiten von Probenbehältern;
- eine Abdeckung (11), die zum Abdecken der Öffnung (12a) ausgebildet ist;
- eine Kühlvorrichtung (14), die zum Kühlen der Probenbehälter ausgebildet ist; und
- eine Kühlvorrichtungssteuerungseinheit (15), die zum Steuern des Betriebes der Kühlvorrichtung (14) ausgebildet ist;
wobei das IVD-Laborsystem zu Folgendem ausgebildet ist:
- Bestimmen, ob die Abdeckung (11) offen ist;
- Bestimmen, ob die Kühlvorrichtungssteuerungseinheit (15) aktiv ist;
- in einem normalen Systemmodus, Abschalten des Betriebes der Aktuatorvorrichtung (10) basierend darauf, dass die Abdeckung (11) offen ist und/oder die Kühlvorrichtungssteuerungseinheit (15) inaktiv ist; und
- in einem Bypass-Systemmodus, Freigeben des Betriebes der Aktuatorvorrichtung (10) basierend darauf, dass die Abdeckung (11) offen ist und die Kühlvorrichtungssteuerungseinheit (15) aktiv ist.

## Revendications

1. Procédé de fonctionnement d'un système de laboratoire de diagnostics in vitro, système de laboratoire de DIV, le système de laboratoire de DIV ayant
- un boîtier (12) avec une ouverture (12a) ;
- un dispositif actionneur (10), agencé dans le boîtier (12), pour le traitement de récipients d'échantillons ;
- un couvercle (11) configuré pour recouvrir l'ouverture (12a) ;
- un dispositif de refroidissement (14) configuré pour refroidir les récipients d'échantillons ; et
- une unité de commande de dispositif de refroidissement (15) configurée pour commander le fonctionnement du dispositif de refroidissement (14) ;
dans lequel le procédé comprend :
- la détermination si le couvercle (11) est ouvert ;
- la détermination si l'unité de commande de dispositif de refroidissement (15) est active ;
- dans un mode de système normal, la désactivation du fonctionnement du dispositif actionneur (10) sur la base du couvercle (11) étant ouvert et/ou de l'unité de commande de dispositif de refroidissement (15) étant inactive ; et
- dans un mode de système de dérivation, l'activation du fonctionnement du dispositif actionneur (10) sur la base du fait que le couvercle (11) est ouvert et que l'unité de commande de dispositif de refroidissement (15) est active.

2. Procédé selon la revendication 1, comprenant en outre :
- dans le mode de système normal, l'activation du fonctionnement du dispositif actionneur (10) sur la base du fait que le couvercle (11) est fermé et que l'unité de commande de dispositif de refroidissement (15) est active ; et
- dans le mode de système de dérivation, la désactivation du fonctionnement du dispositif actionneur (10) sur la base du couvercle (11) étant fermé et/ou de l'unité de commande de dispositif de refroidissement (15) étant inactive.

3. Procédé selon la revendication 1 ou 2, dans lequel le système de laboratoire de DIV comprend un dispositif de verrouillage (13) configuré pour verrouiller le couvercle (11) et le procédé comprend :
- la détermination si le dispositif de verrouillage (13) est verrouillé ; et
- dans le mode de système normal, la désactivation du fonctionnement du dispositif actionneur (10) également sur la base du fait que le dispositif de verrouillage (13) est déverrouillé.

4. Procédé selon l'une des revendications précédentes, comprenant en outre la commutation entre le mode de système normal et le mode de système de dérivation par une opération de l'utilisateur d'un dispositif de commutation (19a) du système de laboratoire de DIV.

5. Procédé selon l'une des revendications précédentes, dans lequel la détermination si l'unité de commande de dispositif de refroidissement (15) est active comprenant la transmission de manière répétée de signaux de battements de cœur (73) à une unité de surveillance (71) du système de laboratoire de DIV et la détermination de l'unité de commande de dispositif de refroidissement (15) comme étant inactive après que le temps depuis la réception d'un dernier signal de battement de cœur par l'unité de surveillance (71) a dépassé un seuil de temporisation.

6. Procédé selon l'une des revendications précédentes, comprenant en outre la commande du dispositif de refroidissement (14) par une seconde unité de commande de dispositif de refroidissement (16) en réaction à la détermination de l'inactivité de l'unité de commande de dispositif de refroidissement (15).

7. Procédé selon la revendication 6, comprenant en outre la commande du dispositif de refroidissement (14) par l'unité de commande de dispositif de refroidissement (15) avec une capacité de refroidissement variable et la commande du dispositif de refroidissement par la seconde unité de commande de dispositif de refroidissement (16) avec une capacité de refroidissement fixe.

8. Procédé selon la revendication 6 ou 7, dans lequel l'unité de commande de dispositif de refroidissement (15) est mise en œuvre par un logiciel et la seconde unité de commande de dispositif de refroidissement (16) est mise en œuvre par un matériel.

9. Procédé selon l'une des revendications précédentes, dans lequel la désactivation du fonctionnement du dispositif actionneur (10) comprend l'arrêt du dispositif actionneur (10) en réaction au fait d'avoir déterminé que le dispositif actionneur (10) est au moins partiellement en mouvement.

10. Procédé selon l'une des revendications précédentes, dans lequel la désactivation du fonctionnement du dispositif actionneur (10) comprend l'interruption d'une alimentation électrique au dispositif actionneur (10).

11. Procédé selon l'une des revendications précédentes, dans lequel le dispositif actionneur (10) comprend au moins l'un parmi un dispositif de transport de récipients d'échantillons, un dispositif de distribution de récipients d'échantillons, un dispositif d'analyse de récipients d'échantillons et un moteur.

12. Procédé selon l'une des revendications précédentes, comprenant en outre, dans le mode de système normal, la désactivation du fonctionnement du dispositif actionneur (10) également sur la base de l'unité de commande de dispositif actionneur (17) configurée pour commander le fonctionnement du dispositif actionneur (10) étant dans un état de réinitialisation de dispositif de commande et/ou de l'unité de commande de dispositif actionneur (17) étant dans un état de gel de dispositif de commande et/ou d'un dispositif d'entrée utilisateur d'urgence (19) étant dans un état actif.

13. Procédé selon l'une des revendications précédentes, comprenant en outre la détermination de la désactivation et de l'activation du fonctionnement du dispositif actionneur (10) en utilisant un circuit logique qui comprend un état de couverture, un état d'unité de commande de dispositif de refroidissement et un état de mode de système comme entrées logiques et un état de dispositif actionneur comme sortie logique.

14. Procédé selon la revendication 13, dans lequel le circuit logique est implémenté dans un circuit électronique en utilisant des composants électroniques.

15. Système de laboratoire de diagnostics in vitro, système de laboratoire de DIV, comprenant :
- un boîtier (12) avec une ouverture (12a) ;
- un dispositif actionneur (10), agencé dans le boîtier (12), pour le traitement de récipients d'échantillons ;
- un couvercle (11) configuré pour recouvrir l'ouverture (12a) ;
- un dispositif de refroidissement (14) configuré pour refroidir les récipients d'échantillons ; et
- une unité de commande de dispositif de refroidissement (15) configurée pour commander le fonctionnement du dispositif de refroidissement (14) ;
dans lequel le système de laboratoire de DIV est configuré pour :
- déterminer si le couvercle (11) est ouvert ;
- déterminer si l'unité de commande de dispositif de refroidissement (15) est active ;
- dans un mode de système normal, désactiver le fonctionnement du dispositif actionneur (10) sur la base du couvercle (11) étant ouvert et/ou de l'unité de commande de dispositif de refroidissement (15) étant inactive ; et
- dans un mode de système de dérivation, activer le fonctionnement du dispositif actionneur (10) sur la base du fait que le couvercle (11) est ouvert et que l'unité de commande de dispositif de refroidissement (15) est active.
